# EUROPÄISCHE PATENTSCHRIFT

(11) **EP 2 855 443 B1**
(45) Veröffentlichungstag und Bekanntmachung des Hinweises auf die Patenterteilung: **30.03.2016**
(21) Anmeldenummer: 13726752.2
(22) Anmeldetag: 29.05.2013
(51) Int. Cl.: C07D 295/088

(54) **VERFAHREN ZUR HERSTELLUNG EINES MONO-N-ALKYL-PIPERAZINS**
PROCESS FOR PREPARING MONO N-ALKYLPIPERAZINE
PROCÉDÉ DE PRODUCTION D'UNE MONO-N-ALKYL-PIPÉRAZINE

(30) Priorität: 01.06.2012 EP 12170580
(43) Veröffentlichungstag der Anmeldung: 08.04.2015
(73) Patentinhaber: BASF SE, 67056 Ludwigshafen (DE)
(72) Erfinder: BOU CHEDID, Roland, 68159 Mannheim (DE); MELDER, Johann-Peter, 67459 Böhl-Iggelheim (DE); ABEL, Ulrich, 67105 Schifferstadt (DE); DOSTALEK, Roman, 67271 Neuleiningen (DE); STEIN, Bernd, 64665 Alsbach-Hähnlein (DE)
(86) Internationale Anmeldenummer: PCT/EP2013/061092
(87) Internationale Veröffentlichungsnummer: WO 2013/178693

(56) Entgegenhaltungen:
- WO-A1-2011/067199
- WO-A1-2012/055893
- NISHIZAWA Y ET AL: "1,4-Bis(2-hydroxyethyl) piperazine prepn. - by dimerisation of di:ethanolamine in presence of copper oxide-chromium oxide-manganese oxide-barium oxide catalyst under hydrogen atmos", WPI / THOMSON,, 29. Juni 1987 (1987-06-29), XP002664153,

## Beschreibung

Die vorliegende Erfindung betrifft ein Verfahren zur Herstellung eines Mono-N-alkyl-piperazins der Formel I 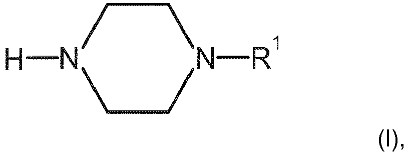 in der R¹ C₁- bis C₅-Alkyl oder 2-(2-Hydroxy-ethoxy)-ethyl bedeutet, durch Umsetzung von Diethanolamin (DEOA) der Formel II 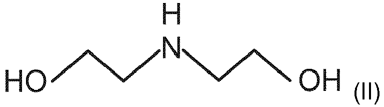 mit einem primären Amin der Formel H₂N-R¹ (III) in Gegenwart von Wasserstoff und eines Katalysator-Formkörpers.

Die Verfahrensprodukte finden u.a. Verwendung als Zwischenprodukte bei der Herstellung von Kraftstoffadditiven (US 3,275,554 A; DE 21 25 039 A und DE 36 11 230 A), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

WO 2011/067199 A1 (BASF SE) betrifft bestimmte aluminiumoxid-, kupfer-, nickel-, kobalt- und zinnhaltige Katalysatoren und ihre Verwendung in Verfahren zur Herstellung eines Amins aus einem primären oder sekundären Alkohol, Aldehyd und/oder Keton. Die Herstellung von N-Methyl-piperazin aus DEOA und Monomethylamin wird auf Seite 25, Zeile 20-21, allgemein erwähnt.

WO 2011/157710 A1 (BASF SE) beschreibt die Herstellung bestimmter zyklischer tertiärer Methylamine, wobei man einen Aminoalkohol aus der Gruppe 1,4-Aminobutanol, 1,5-Aminopentanol, Aminodiglykol (ADG) bzw. Aminoethyl-ethanolamin, mit Methanol bei erhöhter Temperatur in Gegenwart eines kupferhaltigen Heterogenkatalysators in der Flüssigphase umsetzt.

Die WO 2012/049101 A1 (BASF SE) betrifft ein Verfahren zur Herstellung bestimmter zyklischer tertiärer Amine, indem man einen Aminoalkohol aus der Gruppe 1,4-Aminobutanol, 1,5-Aminopentanol, Aminodiglykol (ADG) bzw. Aminoethyl-ethanolamin, mit einem bestimmten primären oder sekundären Alkohol bei erhöhter Temperatur in Gegenwart eines kupferhaltigen Heterogenkatalysators in der Flüssigphase umsetzt.

CN 102 101 847 A (Zhangjiagang Tianyou New Material Techn. Co., Ltd.) beschreibt eine zweistufige Synthese für N-Methyl-N-(2-Chlorethyl)-piperazin aus Aminodiglykol (ADG) via N-Methyl-piperazin als Zwischenprodukt.

CN 102 304 101 A (Shaoxing Xingxin Chem. Co., Ltd.) betrifft die gleichzeitige Herstellung von Piperazin und N-Alkylpiperazinen durch Umsetzung von N-Hydroxyethyl-1,2-ethandiamin mit primären C₁₋₇-Alkoholen in Gegenwart von metallischen Katalysatoren.

EP 446 783 A2 (BASF AG) betrifft u.a. die Herstellung von N-arylsubstituierten Piperazinen durch Aminierung von entsprechenden N,N-Di-(2-hydroxyalkyl)-N-aryl-aminen.

EP 235 651 A1 (BASF AG) lehrt ein Verfahren zur Herstellung von N-Methyl-piperazin aus DE-OA und Methylamin in Gegenwart metallhaltiger Trägerkatalysatoren, insbesondere Cu-haltiger Katalysatoren.

DE 198 59 776 A1 (BASF AG) betrifft bestimmte Aminierungsverfahren unter Einsatz von Katalysator-Formkörpern, die sauerstoffhaltige Verbindungen des Titans und des Kupfers und metallisches Kupfer umfassen.

WO 04/085356 A1 und WO 2010/115759 A2 (beide BASF AG) beschreiben die Verwendung bestimmter Al₂O₃ / Cu / Lanthanoxid - Katalysatoren für die Hydrierung von bestimmten Carbonylverbindungen.

Der vorliegenden Erfindung lag die Aufgabe zugrunde, die Wirtschaftlichkeit bisheriger Verfahren zur Herstellung von Mono-N-alkyl-piperazinen der Formel I zu verbessern und einem Nachteil oder mehreren Nachteilen des Stands der Technik abzuhelfen. Es sollten Bedingungen gefunden werden, die technisch in einfacher Weise herzustellen sind und die es erlauben, das Verfahren mit hohem Umsatz, hoher Ausbeute, Raum-Zeit-Ausbeuten (RZA), Selektivität bei gleichzeitig hoher mechanischer Stabilität des Katalysatorformkörpers und geringer ,Durchgehgefahr', durchzuführen.

[Raum-Zeit-Ausbeuten werden angegeben in ,Produktmenge / (Katalysatorvolumen ● Zeit)' (kg / (I_{Kat}. ● h)) und/oder,Produktmenge / (Reaktorvolumen ● Zeit)' (kg / (I_{Reaktor} ● h)].

Demgemäß wurde ein Verfahren zur Herstellung eines Mono-N-alkyl-piperazins der Formel I 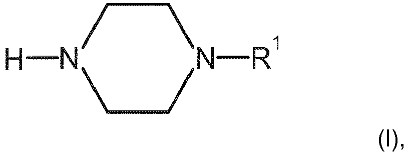 in der R¹ C₁- bis C₅-Alkyl oder 2-(2-Hydroxy-ethoxy)-ethyl bedeutet, durch Umsetzung von Diethanolamin (DEOA) der Formel II 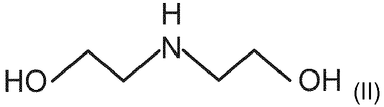 mit einem primären Amin der Formel H₂N-R¹ (III) in Gegenwart von Wasserstoff und eines Katalysator-Formkörpers gefunden, welches dadurch gekennzeichnet ist, dass man die Umsetzung in der Flüssigphase bei einem Absolutdruck im Bereich von 150 bis 250 bar durchführt und die Aminierung mittels eines Katalysator-Formkörpers, dessen Vorläufer gemäß einem Verfahren herstellbar ist, in dem
(i) ein oxidisches Material, umfassend Kupferoxid, Aluminiumoxid und Lanthanoxid bereitgestellt wird,
(ii) dem oxidischen Material pulverförmiges metallisches Kupfer und/oder Kupferblättchen und optional Graphit zugegeben wird,
(iii) das aus ii resultierende Gemisch zu einem Formkörper verformt wird, durchgeführt wird,
wobei das oxidische Material erhältlich ist durch simultanes oder nacheinander erfolgendes Fällen der Komponente Kupferoxid, der Komponente Aluminiumoxid und der Komponente Lanthanoxid und anschließendes Trocknen und Calcinieren
und nach der Verformung nach Schritt iii der Katalysator-Formkörper nochmals calciniert wird.

Bei dem Rest R¹ handelt es sich um 2-(2-Hydroxy-ethoxy)-ethyl oder C₁₋₅-Alkyl, bevorzugt C₁₋₃-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, besonders bevorzugt Methyl, Ethyl und 2-(2-Hydroxy-ethoxy)-ethyl.
Bei dem primären Amin III handelt es sich entsprechend besonders bevorzugt um Monomethylamin, Monoethylamin oder 1-Amino-2-(2-hydroxy-ethoxy)-ethan (Aminodiglykol, ADG).

Mit dem erfindungsgemäßen Verfahren bevorzugt herstellbar sind Amine der Formel I 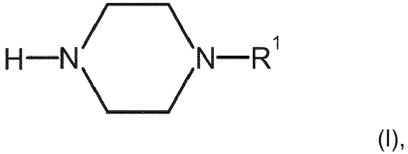 in der R¹ = Methyl, Ethyl oder 2-(2-Hydroxy-ethoxy)-ethyl bedeutet.

Insbesondere werden Katalysator-Förmkörper eingesetzt, die dadurch gekennzeichnet sind, dass das oxidische Material
(a) Kupferoxid mit einem Anteil im Bereich von 50 ≤ x ≤ 80 Gew.-%, vorzugsweise 55 ≤ x ≤ 75 Gew.-%, jeweils berechnet als CuO,
(b) Aluminiumoxid mit einem Anteil im Bereich von 15 ≤ y ≤ 35 Gew.-%, vorzugsweise 20 ≤ y ≤ 30 Gew.-%, und
(c) Lanthanoxid mit einem Anteil im Bereich von 2 ≤ z ≤ 20 Gew.-%, bevorzugt 3 ≤ z ≤ 15 Gew.-%, weiter bevorzugt 3,5 ≤ z ≤ 10 Gew.-%,
jeweils bezogen auf das Gesamtgewicht des oxidischen Materials nach Calcinierung, wobei gilt: 80 ≤ x + y + z ≤ 100, insbesondere 95 ≤ x + y + z ≤ 100, umfasst.

Das Verfahren kann kontinuierlich oder diskontinuierlich durchgeführt werden. Bevorzugt ist eine kontinuierliche Fahrweise.

In der Kreisgasfahrweise werden die Ausgangsstoffe (DEOA, das primäre Amin III) in einem Kreisgasstrom verdampft und gasförmig dem Reaktor zugeführt.

Die Edukte (DEOA, das primäre Amin III) können auch als wässrige Lösungen verdampft und mit dem Kreisgasstrom auf das Katalysatorbett geleitet werden.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Alternativ erfolgt die Umsetzung vorteilhaft in einem Rohrbündelreaktor oder in einer Monostranganlage.

Bei einer Monostranganlage kann der Rohrreaktor, in dem die Umsetzung erfolgt, aus einer Hintereinanderschaltung mehrerer (z. B. zweier oder dreier) einzelner Rohrreaktoren bestehen. Optional ist hier vorteilhaft eine Zwischeneinspeisung von Feed (enthaltend das DEOA und/oder primäres Amin III und/oder H₂) und/oder Kreisgas und/oder Reaktoraustrag aus einem nachgeschalteten Reaktor möglich.

Die Kreisgasmenge liegt bevorzugt im Bereich von 40 bis 1500 m³ (bei Normaldruck) / [m³ Katalysator (Schüttvolumen) • h], insbesondere im Bereich von 400 bis 1400 m³ (bei Normaldruck) / [m³ Katalysator (Schüttvolumen) • h]. (Normaldruck = 1 bar abs.)

Das Kreisgas enthält bevorzugt mindestens 10, besonders 50 bis 100, ganz besonders 80 bis 100, Vol.% Wasserstoff (H₂).

In bevorzugten Ausführungsformen werden im erfindungsgemäßen Verfahren die Katalysator-Formkörper als Voll,- Tränk-, Schalen- oder Fällkatalysatoren eingesetzt.

Der in dem erfindungsgemäßen Verfahren verwendete Katalysator für die Aminierung zeichnet sich dadurch aus, dass die Komponente Kupferoxid, die Komponente Aluminiumoxid und die Komponente Lanthanoxid, bevorzugt mit einer Sodalösung, simultan oder nacheinander gefällt werden, im Anschluss getrocknet, calciniert, verformt, z.B. tablettiert, und nochmals calciniert wird.

Kupferoxid bedeutet CuO, Cu₂O oder ein Gemisch aus beiden Oxiden. Bei Mengenangaben wird Kupfer-(I)-oxid als Kupfer-(II)-oxid berechnet.
Aluminiumoxid bedeutet Al₂O₃ und Lanthanoxid bedeutet La₂O₃.

Insbesondere kommt folgende Fällungsmethode in Betracht:
A) Eine Kupfersalzlösung, eine Aluminiumsalzlösung und eine Lösung eines Salzes des Lanthans oder eine Lösung, enthaltend Kupfer-, Aluminium- und Lanthan-Salz, wird simultan bzw. werden nacheinander mit einer Sodalösung gefällt.
B) Fällung einer Kupfersalzlösung und, separat, einer Lösung eines Salzes des Lanthans oder einer Lösung, enthaltend Kupfersalz und ein Salz des Lanthans, auf einen vorgefertigten Aluminiumoxidträger. Dieser liegt in einer besonders bevorzugten Ausführungsform als Pulver in einer wässrigen Suspension vor. Das Trägermaterial kann aber z.B. auch als Kugeln, Stränge, Splitt oder Tabletten vorliegen.
   In einer besonderen Ausführungsform zu B) (B1) wird eine Kupfersalzlösung und eine Lösung eines Salzes des Lanthans oder eine Lösung, enthaltend Kupfersalz und ein Salz des Lanthans, bevorzugt mit Sodalösung, gefällt. Als Vorlage wird eine wässrige Suspension des Trägermaterials Aluminiumoxid verwendet.

Ausgefällte Niederschläge, die aus A) oder B) resultieren, werden in üblicher Weise abgetrennt, z.B. filtriert, und vorzugsweise alkalifrei gewaschen, wie dies beispielsweise in der DE 198 09 418 A1 (BASF AG) beschrieben ist.

Nach der Fällung der Komponenten, besonders die Endprodukte aus A) oder aus B), werden diese bei erhöhter Temperatur, besonders bei Temperaturen von 50 bis 150 °C, vorzugsweise bei 110 bis 130 °C, getrocknet (z.B. über einen Zeitraum von 5 bis 30 Stunden, bevorzugt 10 bis 20 Stunden) und im Anschluss, vorzugsweise z.B. über einen Zeitraum von 0,5 bis 6 Stunden, besonders 1 bis 3 Stunden, bei im Allgemeinen 200 bis 700 °C, insbesondere bei 400 bis 650 °C, calciniert.

Als Ausgangssubstanzen für A) und/oder B) können prinzipiell alle in den bei der Fällung verwendeten Lösungsmitteln (bevorzugt ist Wasser) löslichen Cu(I)- und/oder Cu(II)-Salze, wie beispielsweise Nitrate, Carbonate, Acetate, Oxalate oder Ammonium-Komplexe, sowie analoge Aluminiumsalze und Salze des Lanthans verwendet werden. Besonders bevorzugt wird als Kupfersalz Kupfer-(II)-nitrat eingesetzt. Als Lanthansalz wird bevorzugt Lanthannitrat eingesetzt. Als Aluminiumsalz wird bevorzugt Aluminiumnitrat eingesetzt.

Die Zusammensetzung des oxidischen Materials ist bevorzugt so beschaffen, dass der Anteil an Kupferoxid im Bereich von 50 bis 80 Gew.-%, besonders 55 bis 75 Gew.-%, jeweils berechnet als CuO, der Anteil an Lanthanoxid im Bereich von 2 bis 20 Gew.-%, besonders 3 bis 15 Gew.-%, und der Anteil an Aluminiumoxid im Bereich 15 bis 35 Gew.-%, besonders 20 bis 30 Gew.-%, für alle Komponenten jeweils bezogen auf das Gesamtgewicht der Summe der oben genannten oxidischen Bestandteile, liegt, wobei diese drei Oxide zusammen mindestens 80 Gew.-%, besonders mindestens 95 Gew.-%, des oxidischen Materials nach Calcinierung darstellen, wobei gegebenfalls zugefügter Zement, z.B. Tonerdezement, nicht dem oxidischen Material in obigem Sinne zugerechnet wird.

In einer bevorzugten Ausführungsform betrifft die vorliegende Erfindung daher ein Verfahren, wie oben beschrieben, das dadurch gekennzeichnet ist, dass das oxidische Material
(a) Kupferoxid mit einem Anteil im Bereich von 50 ≤ x ≤ 80 Gew.-%, vorzugsweise 55 ≤ x ≤ 75 Gew.-%, jeweils berechnet als CuO,
(b) Aluminiumoxid mit einem Anteil im Bereich von 15 ≤ y ≤ 35 Gew.-%, vorzugsweise 20 ≤ y ≤ 30 Gew.-%, und
(c) Lanthanoxid mit einem Anteil im Bereich von 2 ≤ z ≤ 20 Gew.-%, bevorzugt 3 ≤ z ≤ 15 Gew.-%, weiter bevorzugt 2 ≤ z ≤ 10 Gew.-%,
jeweils bezogen auf das Gesamtgewicht des oxidischen Materials nach Calcinierung, wobei gilt: 80 ≤ x + y + z ≤ 100, insbesondere 95 ≤ x + y + z ≤ 100, umfasst.

Die im erfindungsgemäßen Verfahren eingesetzten Katalysatoren zeichnen sich auch dadurch aus, dass die Zugabe des Lanthansalzes bei der Fällung zu einer hohen Stabilität des schließlich resultierenden Formkörpers, der als Katalysator eingesetzt wird, führt.

Dem oxidischen Material wird/werden anschließend (Schritt ii) pulverförmiges Kupfer und/oder Kupferblättchen und optional Graphit zugegeben. Bevorzugt wird pulverförmiges Kupfer und Graphit zugegeben. Die Zugabe von Graphit kann auch vor der Kupferzugabe erfolgen, wobei dann bevorzugt zunächst eine Vorkompaktierung durchgeführt wird. Z.B. wird Graphit in Mengen im Bereich von 0 bis 5 Gew.-%, bevorzugt im Bereich von 0,5 bis 4 Gew.-%, besonders bevorzugt im Bereich von 0,8 bis 2 Gew.-%, jeweils bezogen auf das Gesamtgewicht des oxidischen Materials nach Calicinierung, zugesetzt.

Als pulverförmiges Kupfer wird bevorzugt solches eingesetzt, welches einen Korndurchmesser im Bereich von 1 bis 700 µm, vorzugsweise im Bereich von 5 bis 500 µm, aufweist. Besonders bevorzugt wird ein pulverförmiges Kupfer eingesetzt, bei dem die Siebanalyse einen Anteil an Partikeln > 500 µm von ≤ 6 %, besonders einen Anteil an Partikeln > 350 µm von ≤ 5 %, ergibt. Die Korn-Morphologie ist bevorzugt kugelförmig.

Als Kupferblättchen werden bevorzugt solche eingesetzt, die einen D 50 - Wert im Bereich von 5 bis 40 µm, besonders im Bereich von 10 bis 35 µm, aufweisen (,D 50 - Wert' bedeutet, dass 50 % der Partikel kleiner sind als der angegebene Wert). Bevorzugt ergibt die Siebanalyse einen Anteil an Partikeln > 45 µm von ≤ 6 %, besonders ≤ 2 %. Die Kupferblättchen weisen bevorzugt eine lamellare Blättchenstruktur auf.

Bevorzugt werden pulverförmiges Kupfer und/oder Kupferblättchen zusammengenommen in Mengen im Bereich von 0,5 bis 40 Gew.-%, bevorzugt im Bereich von 2 bis 20 Gew.-%, besonders bevorzugt im Bereich von 3 bis 10 Gew.-%, jeweils bezogen auf das Gesamtgewicht des oxidischen Materials nach Calicinierung, zugesetzt.

In besonderen Ausführungsformen kann das oxidische Material in einem Anteil von höchstens 10 Gew.-%, bevorzugt höchstens 5 Gew.-%, bezogen auf das Gesamtgewicht des oxidischen Materials nach Calcinierung, mindestens eine weitere Komponente aufweisen, die ausgewählt wird aus der Gruppe bestehend aus Oxiden der Elemente Re, Fe, Ru, Co, Rh, Ir, Ni, Pd und Pt.

Bevorzugt enthält der Katalysator-Formkörper kein Rhenium, kein Ruthenium, kein Eisen und/oder kein Zink, jeweils weder in metallischer (Oxidationsstufe = 0) noch in einer ionischen (Oxidationsstufe ≠ 0), insbesondere oxidierten, Form.

Bevorzugt enthält der Katalysator-Formkörper kein Silber und/oder Molybdän, jeweils weder in metallischer (Oxidationsstufe = 0) noch in einer ionischen (Oxidationsstufe ≠ 0), insbesondere oxidierten, Form.

Bevorzugt enthält der Katalysator-Formkörper keine sauerstoffhaltigen Verbindungen des Siliziums, Zirkoniums und/oder des Chroms.

Bevorzugt enthält der Katalysator-Formkörper keine sauerstoffhaltigen Verbindungen des Titans.

In Schritt iii wird das aus Schritt ii resultierende Gemisch zu einem Formkörper verformt und danach calciniert. Bevorzugt wird dem Gemisch vor dem Verformen zum Formkörper Graphit zugesetzt. Vorzugsweise wird so viel Graphit zugegeben, dass die Verformung zu einem Formkörper besser durchgeführt werden kann. In einer bevorzugten Ausführungsform werden 0,5 bis 5 Gew.-%, besonders 1 bis 3 Gew.-%, Graphit, bezogen auf das Gesamtgewicht des aus Schritt ii resultierende Gemisches, zugegeben.

Die Summe der Anteile aus oxidischem Material, metallischem Kupferpulver und/oder Kupferblättchen und ggf. Graphit ergibt bevorzugt mindestens 95 Gew.-%, besonders mindestens 98 Gew.-%, des Katalysator-Formkörpers.

Die Verformung in Schritt iii führt bevorzugt zu Tabletten, Ringen, Ringtabletten, Extrudaten, Wabenkörpern oder ähnlichen Formkörpern. Hierfür eignen sich sämtliche aus dem Stand der Technik bekannte Verfahren.

Im Anschluss an die Verformung erhaltene Formkörper werden nochmals, mindestens einmal, calciniert. Die Calcinierung erfolgt jeweils bevorzugt über eine Zeit von im allgemeinen 0,5 bis 10 Stunden (h), besonders 0,5 bis 2,5 Stunden. Die Temperatur bei diesem mindestens einen Calcinierungsschritt (und auch bei den optionalen Wiederholungs-Calcinierungsschritten) liegt im allgemeinen im Bereich von 200 bis 600 °C, bevorzugt im Bereich von 250 bis 500 °C und besonders bevorzugt im Bereich von 270 bis 400 °C.

In einer weiteren Ausführung kann der erhaltene Formkörper auch noch mit siedendem Wasser und/oder Wasserdampf behandelt werden, bevor er für die Aminierung eingesetzt wird.

Bei Einsatz als Katalysator in der oxidischen Form wird der Formkörper vor Beschickung mit den Edukten mit reduzierenden Gasen, beispielsweise Wasserstoff, vorzugsweise Wasserstoff/Inertgas-Gemischen, insbesondere Wasserstoff/Stickstoff-Gemischen, bei erhöhten Temperaturen, z.B. im Bereich von 100 bis 500 °C, bevorzugt im Bereich von 150 bis 350 °C und insbesondere im Bereich von 180 bis 200 °C, vorreduziert. Bevorzugt wird dabei ein Gasgemisch mit einem Wasserstoffanteil im Bereich von 1 bis 100 Vol.-%, besonders bevorzugt im Bereich von 1 bis 50 Vol.-%, verwendet.

In einer bevorzugten Ausführungsform wird der Formkörper vor dem Einsatz als Katalysator in an sich bekannter Weise durch Behandlung mit reduzierenden Medien aktiviert. Das Aktivieren erfolgt entweder vorab in einem Reduktionsofen oder nach dem Einbau im Reaktor. Ist der Reaktor vorab im Reduktionsofen aktiviert worden, wird er in den Reaktor eingebaut und direkt unter Wasserstoffdruck mit den Edukten beschickt.

Das erfindungsgemäße Verfahren wird bevorzugt kontinuierlich durchgeführt, wobei der Katalysator bevorzugt als Festbett im Reaktor angeordnet ist. Dabei ist sowohl eine Anströmung des Katalysatorfestbetts von oben als auch von unten möglich.

Das primäre Amin III wird bevorzugt in der 0,5- bis 20-fachen molaren Menge, weiter bevorzugt in der 2- bis 17-fachen molaren Menge, besonders in der 5- bis 15-fachen molaren Menge, insbesondere in der 6- bis 14-fachen molaren Menge, weiter besonders in der 7- bis 13-fachen molaren Menge, ganz besonders in der 8- bis 12-fachen molaren Menge, weiter ganz besonders in der 8- bis 10-fachen molaren Menge, jeweils bezogen auf das eingesetzte DEOA, eingesetzt.
Besonders bevorzugt wird im Fall von Aminodiglykol (ADG) als primärem Amin III das primäre Amin in der 0,5- bis 2-fachen, insbesondere in der 0,6- bis 1,2-fachen, molaren Menge, jeweils bezogen auf das eingesetzte DEOA, eingesetzt.
Besonders bevorzugt wird im Fall von Monomethylamin (MMA) als primärem Amin III das primäre Amin in der 4- bis 13-fachen, insbesondere in der 5- bis 12-fachen, molaren Menge, jeweils bezogen auf das eingesetzte DEOA, eingesetzt.

Besonders bevorzugt wird im Fall von Monoethylamin (MEA) als primärem Amin III das primäre Amin in der 2- bis 10-fachen, insbesondere in der 3- bis 9-fachen, molaren Menge, jeweils bezogen auf das eingesetzte DEOA, eingesetzt.

Das primäre Amin III kann als wässrige Lösung, besonders als 30 bis 95 Gew.-%ige wässrige Lösung, z. B. auch 65 bis 90 Gew.-%ige wässrige Lösung, eingesetzt werden. Monomethylamin und Monoethylamin werden bevorzugt auch ohne weiteres Lösungsmittel (Druckgas, Reinheit besonders 95 bis 100 Gew.-%ig) eingesetzt.

Das Edukt DEOA wird bevorzugt als wässrige Lösung, besonders als 75 bis 95 Gew.-%ige wässrige Lösung, z.B. als 80 bis 85 Gew.-%ige wässrige Lösung, eingesetzt.

Bevorzugt wird eine Abgasmenge von 5 bis 800 Normkubikmeter / (Kubikmeter Katalysator • h), insbesondere 20 bis 300 Normkubikmeter / (m³ Katalysator • h), gefahren. [Normkubikmeter = auf Normalbedingungen (20 °C, 1 bar abs.) umgerechnetes Volumen].
Katalysatorvolumen-Angaben beziehen sich immer auf das Schüttvolumen.

Die Aminierung der primären Alkoholgruppen des Edukts DEOA wird in der Flüssigphase durchgeführt. Bevorzugt ist das Festbettverfahren in der Flüssigphase.

Beim kontinuierlichen Festbettverfahren in der Flüssigphase ist folgende Verfahrensausgestaltung, die sich u.a. vorteilhaft auf die Katalysatorperformance auswirkt, besonders bevorzugt. Man leitet die Edukte (DEOA, primäres Amin III) inklusive Wasserstoff zunächst bei einer Temperatur im Bereich von 80 bis 160 °C, bevorzugt 100 bis 140 °C, besonders bevorzugt 110 bis 130 °C, über den Katalysator und danach, z.B. nach 1 bis 240 Min., bevorzugt 5 bis 120 Min., besonders bevorzugt 10 bis 90 Min, weiter besonders bevorzugt 20 bis 60 Min., erhöht man die Temperatur auf 180 bis 240 °C, besonders 180 bis 235 °C, bevorzugt 185 bis 230 °C, insbesondere 190 bis 225 °C. Demnach ist also eine Prozedur zum Anfahren bei niedrigeren Temperaturen vorgeschaltet. Das aus der Anfahrprozedur resultierende Umsetzungsprodukt kann verworfen oder in die Umsetzung zurückgeführt werden.

Beim Arbeiten in der Flüssigphase leitet man die Edukte (DEOA, primäres Amin III), bevorzugt simultan, in flüssiger Phase bei Drücken von 15,0 bis 25,0 MPa (150 bis 250 bar), bevorzugt 15,5 bis 23,0 MPa, weiter bevorzugt 16,0 bis 22,0 MPa, weiter bevorzugt 16,5 bis 21,5 MPa, besonders bevorzugt 17,0 bis 21,0 MPa, und Temperaturen von im Allgemeinen 180 bis 240 °C, besonders 180 bis 235 °C, bevorzugt 185 bis 230 °C, insbesondere 190 bis 225 °C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im Allgemeinen im Bereich von 0,2 bis 0,8, bevorzugt 0,3 bis 0,7, besonders bevorzugt 0,4 bis 0,6, weiter bevorzugt 0,4 bis 0,5, kg DEOA pro Liter Katalysator (Schüttvolumen) und Stunde (DEOA berechnet als 100 %ig). Gegebenenfalls kann eine Verdünnung der Edukte mit einem geeigneten Lösungsmittel, wie Wasser, Tetrahydrofuran, Dioxan, N-Methylpyrrolidon oder Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Die Umsetzung wird bevorzugt bei einer Katalysatorbelastung im Bereich von 100 bis 1500 Normliter Wasserstoff / (I_{Kat}. ● h), besonders einer Katalysatorbelastung im Bereich von 400 bis 1400 Normliter Wasserstoff / (I_{Kat.} ● h), durchführt.
[Normliter = NI = auf Normalbedingungen (20 °C, 1 bar abs.) umgerechnetes Volumen].

Der Druck im Reaktionsgefäß, welcher sich aus der Summe der Partialdrücke des primären Amins III, des DEOAs und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Bei dem kontinuierlichen Arbeiten in der Flüssigphase kann das überschüssige primäre Amin III zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe) wirkt sich im Allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z. B. durch eine fraktionierende Rektifikation. Geeignete Aufarbeitungsverfahren sind z. B. in EP 1 312 600 A und EP 1 312 599 A (beide BASF AG) beschrieben. Das überschüssige primäre Amin und der Wasserstoff werden vorteilhaft wieder in die Reaktionszone zurückgeführt. Das gleiche gilt für die eventuell nicht vollständig umgesetztes DEOA.

Eine Aufarbeitung des Produkts der Umsetzung ist bevorzugt wie folgt ausgestaltet:
Aus dem Reaktionsprodukt der Umsetzung werden durch Destillation
   (i) zunächst ggf. unumgesetztes primäres Amin III, R¹ bevorzugt = C₁- bis C₅-Alkyl, über Kopf abgetrennt,
   (ii) Wasser über Kopf abgetrennt,
   (iii) ggf. vorhandene Nebenprodukte mit einem niedrigeren Siedepunkt als dem des Verfahrensprodukts I (Niedersieder) über Kopf abgetrennt,
   (iv) das Verfahrensprodukt Mono-N-alkyl-piperazin I über Kopf abgetrennt, wobei ggf. vorhandene Nebenprodukte mit einem höheren Siedepunkt als dem des Verfahrensprodukts I (Hochsieder) und ggf. vorhandenes unumgesetztes DEOA (II) im Sumpf verbleiben.
Bei der Umsetzung des erfindungsgemäßen Verfahrens kann als Nebenprodukt das Alkylaminoethylethanolamin der Formel IV 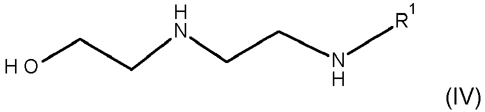 entstehen: 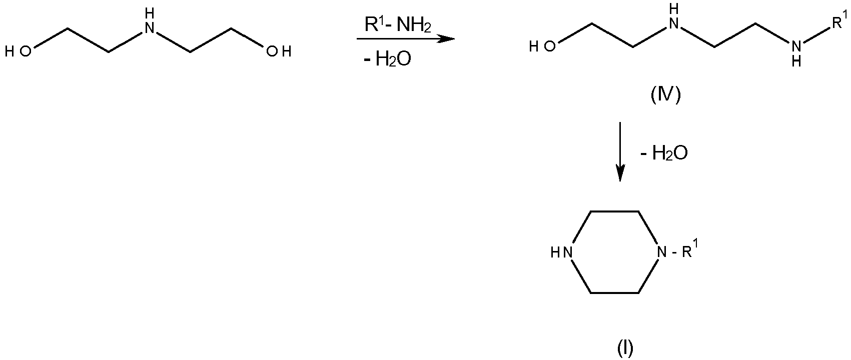
Deshalb werden im Besonderen durch Destillation
   (v) aus dem Sumpf des Schrittes iv ggf. vorhandenes unumgesetztes DEOA (II) und/oder ggf. vorhandenes Alkylaminoethylethanolamin als Nebenprodukt mit der Formel IV über Kopf abgetrennt und in die Umsetzung zurückgeführt.

In Schritt i abgetrenntes primäres Amin III mit einer Reinheit von 90 bis 99,9 Gew.-%, besonders 95 bis 99,9 Gew.-%, wird bevorzugt in die Umsetzung zurückgeführt, wobei weiter bevorzugt ein Teil des abgetrennten Amins III, besonders 1 bis 30 Gew.-% des abgetrennten Amins III, weiter besonders 2 bis 25 Gew.-% des abgetrennten Amins III, ausgeschleust wird.

Eine Aufarbeitung des Produkts der Umsetzung von Aminodiglykol (ADG), also R¹ = 2-(2-Hydroxy-ethoxy)-ethyl, mit DEOA ist bevorzugt wie folgt ausgestaltet:
Aus dem Reaktionsprodukt der Umsetzung werden durch Destillation
   (i) zunächst Wasser über Kopf abgetrennt,
   (ii) ggf. unumgesetztes ADG über Kopf abgetrennt,
   (iii) ggf. vorhandene Nebenprodukte mit einem niedrigeren Siedepunkt als dem des Verfahrensprodukts I (Niedersieder) über Kopf abgetrennt,
   (iv) das Verfahrensprodukt Mono-N-alkyl-piperazin I über Kopf abgetrennt, wobei ggf. vorhandene Nebenprodukte mit einem höheren Siedepunkt als dem des Verfahrensprodukts I (Hochsieder) und ggf. vorhandenes unumgesetztes DEOA (II) im Sumpf verbleiben.
Im Besonderen werden durch Destillation
   (v) aus dem Sumpf des Schrittes iv ggf. vorhandenes unumgesetztes DEOA (II) und/oder ggf. vorhandenes Alkylaminoethylethanolamin als Nebenprodukt mit der Formel IV über Kopf abgetrennt und in die Umsetzung zurückgeführt.

In Schritt ii abgetrenntes ADG mit einer Reinheit von 90 bis 99,9 Gew.-%, besonders 95 bis 99,9 Gew.-%, wird bevorzugt in die Umsetzung zurückgeführt, wobei weiter bevorzugt ein Teil des abgetrennten ADG, besonders 1 bis 30 Gew.-% des abgetrennten ADG, weiter besonders 5 bis 25 Gew.-% des abgetrennten ADG, ausgeschleust wird.

Alle Druckangaben beziehen sich auf den Absolutdruck.

Alle ppm-Angaben beziehen sich auf die Masse.

### Beispiele

### 1. Herstellung des Katalysators A

Ein Gemisch aus 13,39 kg einer 19,34 %igen Kupfer-(II)-nitratlösung und 14,78 kg einer 8,12 %igen Aluminiumnitratlösung und 0,56 kg einer 37,58 %igen Lanthannitratlösung (Einsatzstoff: Lanthan-(III)-nitrat • 6 H₂O) wurden in 1,5 I Wasser gelöst (Lösung 1). Lösung 2 waren 60 kg einer 20 %igen Natriumcarbonatlösung (Einsatzstoff: Na₂CO₃ wasserfrei). Lösung 1 und Lösung 2 wurden über getrennte Leitungen in ein Fällgefäß, das mit einem Rührer versehen war und 10 I auf 60 °C erhitztes Wasser enthielt, geleitet. Hierbei wurde durch entsprechende Einstellung der Zufuhrgeschwindigkeiten der Lösung 1 und Lösung 2 der pH-Wert auf 6,0 gebracht.

Unter Konstanthaltung des pH-Wertes bei 6,0 und der Temperatur bei 60 °C wurde die gesamte Lösung 1 mit der Sodalösung zur Reaktion gebracht. Die so gebildete Suspension wurde anschließend auf 80 °C erhitzt, der pH-Wert durch Zugabe von verdünnter Sodalösung (Lösung 2) auf 8,0 erhöht und 15 Min. bei diesem pH und bei 80 °C nachgerührt. Die Suspension wurde filtriert und mit destilliertem Wasser so lange gewaschen, bis der Nitratgehalt des Waschwassers < 10 ppm betrug.

Der Filterkuchen wurde 16 h lang bei 120 °C getrocknet und anschließend 2 h lang bei 600 °C calciniert. Das so erhaltene Katalysatorpulver wurde mit 1 Gew.-% Graphit vorkompaktiert. Das erhaltene Kompaktat wurde mit 5 Gew.-% Cu-Blättchen, die einen D 50 - Wert im Bereich von 5 bis 40 µm aufwiesen (z.B. erhältlich von der Firma Schlenk Metallpulver GmbH & Co. KG, D-91154 Roth-Barnsdorf), und anschließend mit 2 Gew.-% Graphit gemischt und zu Tabletten von 3 mm Durchmesser und 3 mm Höhe verpresst. Die Tabletten wurden schließlich 2 h lang bei 350 °C calciniert.

Der so hergestellte Katalysator hatte die chemische Zusammensetzung 61,5 Gew.-% CuO / 28,5 Gew.-% Al₂O₃ / 5,0 Gew.-% La₂O₃ / 5 Gew.-% Cu, (Graphitanteil herausgerechnet).

### 2. Umsetzung von DEOA mit Monomethylamin (MMA) in einem kontinuierlich betriebenen Rohrreaktor

Ein beheizter Rohrreaktor mit 14 mm Innendurchmesser, einem zentral angebrachten Thermoelement und einem Gesamtvolumen von 1000 ml wurde im unteren Teil mit einer Schicht Glaskugeln (250 ml) befüllt, darüber mit 500 ml des Katalysators A und schließlich der restliche Teil wiederum mit Glaskugeln befüllt. Vor der Reaktion wurde der Katalysator bei max. 200 °C unter Wasserstoff (25 Nl/h) (NI = Normliter = auf Normalbedingungen (20 °C, 1 bar abs.) umgerechnetes Volumen) bei Normaldruck 24 Stunden aktiviert. Durch den Reaktor wurden von unten nach oben 300 g/h DEOA (85 %ig wässrig), 600 g/h des primären Amins und 200 Nl/h Wasserstoff dosiert. Der Reaktor wurde bei einer Temperatur von ca. 185 bis 220 °C und einem Gesamtdruck von 200 bar gehalten. Die Reaktionstemperatur wurde so gewählt, dass ein DEOA-Umsatz von > 90 % erreicht wurde. Das aus dem Reaktor austretende Gemisch wurde abgekühlt und auf Normaldruck entspannt. Zu verschiedenen Zeitpunkten wurden Proben vom Reaktionsgemisch genommen und mittels Gaschromatographie analysiert. Hierfür wurde eine 30 m lange GC Säule "RTX-5 Amine" verwendet, mit einem Temperaturprogramm: 70 °C/5 Min., aufheizen auf 280 °C mit einer Geschwindigkeit von 5 °C/Min., bei 280 °C/10 Minuten.
Die Ergebnisse der Versuche sind der folgenden Tabelle I zu entnehmen.

**Tabelle I**

| Kat. | Druck bar | H₂ NI / (l•h) | MV MMA:DEOA mol/mol | Temp. °C | Zulauf DEOA *) | Belastung ber. 100 %ig DEOA kg / (l•h) | Umsatz DEOA mol% | Sel. NMePIP bez. auf DEOA mol% |
|---|---|---|---|---|---|---|---|---|
| A | 120 | 400 | 8 | 195 | 85 %ig | 0,5 | 98 | 73 |
| A | 200 | 400 | 8 | 195 | 85 %ig | 0,5 | 97 | 88 |
| A | 200 | 400 | 12 | 195 | 85 %ig | 0,5 | 97 | 89 |
| A | 200 | 400 | 5 | 195 | 85 %ig | 0,5 | 98 | 68 |

| | | | | | | | | |
|---|---|---|---|---|---|---|---|---|
| Kat.: Katalysator Temp.: Temperatur im Reaktor Bel.: Katalysatorbelastung [kg DEOA / (Liter_{Kat}. • h)] MV: Molverhältnis im Feed Sel.: Selektivität NMePIP: Monomethylpiperazin (N-Methyl-PIP) *): wässrige Lösung, in Gew.-% | | | | | | | | |

### 3. Umsetzung von DEOA mit Monoethylamin (MEA) in einem kontinuierlich betriebenen Rohrreaktor

Ein beheizter Rohrreaktor mit 14 mm Innendurchmesser, einem zentral angebrachten Thermoelement und einem Gesamtvolumen von 1000 ml wurde im unteren Teil mit einer Schicht Glaskugeln (250 ml) befüllt, darüber mit 500 ml des Katalysators A und schließlich der restliche Teil wiederum mit Glaskugeln befüllt. Vor der Reaktion wurde der Katalysator bei max. 200 °C unter Wasserstoff (25 Nl/h) [NI = Normliter = auf Normalbedingungen (20 °C, 1 bar abs.) umgerechnetes Volumen] bei Normaldruck 24 Stunden aktiviert. Durch den Reaktor wurden von unten nach oben 180 g/h DEOA (85 %ig wässrig), 460 g/h des primären Amins und 200 Nl/h Wasserstoff dosiert. Der Reaktor wurde bei einer Temperatur von ca. 185 bis 220 °C und einem Gesamtdruck von 200 bar gehalten. Die Reaktionstemperatur wurde so gewählt, dass ein DEOA-Umsatz von > 90 % erreicht wurde. Das aus dem Reaktor austretende Gemisch wurde abgekühlt und auf Normaldruck entspannt. Zu verschiedenen Zeitpunkten wurden Proben vom Reaktionsgemisch genommen und mittels Gaschromatographie analysiert. Hierfür wurde eine 30 m lange GC Säule "RTX-5 Amine" verwendet, mit einem Temperaturprogramm: 70 °C/5 Min., aufheizen auf 280 °C mit einer Geschwindigkeit von 5 °C/Min., bei 280 °C/10 Minuten.
Die Ergebnisse der Versuche sind der folgenden Tabelle II zu entnehmen.

**Tabelle II**

| Kat. | Druck bar | H₂ NI / (l•h) | MV MEA:DEOA mol/mol | Temp. °C | Zulauf DEOA *) | Belastung ber. 100 %ig DEOA kg / (l•h) | Umsatz DEOA mol% | Sel. NEt-PIP bez. auf DEOA mol% |
|---|---|---|---|---|---|---|---|---|
| A | 200 | 400 | 5 | 217 | 85 %ig | 0,4 | 85 | 33 |
| A | 200 | 200 | 5 | 214 | 85 %ig | 0,4 | 93 | 39 |
| A | 200 | 200 | 3 | 202 | 85 %ig | 0,4 | 79 | 31 |
| A | 200 | 100 | 5 | 200 | 85 %ig | 0,2 | 95 | 44 |
| A | 200 | 100 | 9 | 211 | 85 %ig | 0,2 | 89 | 40 |
| A | 200 | 400 | 5 | 193 | 85 %ig | 0,3 | 82 | 35 |

| | | | | | | | | |
|---|---|---|---|---|---|---|---|---|
| Kat.: Katalysator Temp.: Temperatur im Reaktor Bel.: Katalysatorbelastung [kg DEOA / (Liter_{Kat.} • h)] MV: Molverhältnis im Feed Sel.: Selektivität NEtPIP: Monoethylpiperazin (N-Ethyl-PIP) *) wässrige Lösung, in Gew.-% | | | | | | | | |

### 4. Umsetzung von DEOA mit Aminodiglykol (ADG, 1-Amino-2-(2-hydroxy-ethoxy)-ethan) in einem Batchreaktor

Ein Batchreaktor mit Rührer, einem Thermoelement und einem Gesamtvolumen von 300 ml wurde gefüllt mit 7,5 g aktiviertem Katalysator. Dazu wurde der Katalysator bei max. 200 °C unter Wasserstoff [25 Nl/h) (NI = Normliter = auf Normalbedingungen (20 °C, 1 bar abs.) umgerechnetes Volumen) bei Normaldruck 24 Stunden aktiviert. Das Eduktgemisch von DEOA und ADG wurde vorgelegt und der Reaktor wurde aufgeheizt auf 180 °C. Das gesamte Reaktionsgemisch wurde dann mit 200 bar Wasserstoff beaufschlagt. Zu verschiedenen Zeitpunkten wurden Proben vom Reaktionsgemisch genommen und mittels Gaschromatographie analysiert. Hierfür wurde eine 30 m lange GC Säule "RTX-5 Amine" verwendet, mit einem Temperaturprogramm: 70 °C/5 Min., aufheizen auf 280 °C mit einer Geschwindigkeit von 5 °C/Min., bei 280 °C/10 Minuten.
Die Ergebnisse der Versuche sind der folgenden Tabelle III zu entnehmen.

**Tabelle III**

| Kat. | Druck bar | Temp. °C | Zeit (h) | DEOA (g) | MV ADG:DEOA mol/mol | Umsatz DEOA | Umsatz ADG | Sel.. HE-OEtPIP bez. DEOA (mol%) | Sel. HEOEtPIP bez. ADG (mol%) |
|---|---|---|---|---|---|---|---|---|---|
| A | 200 | 180 | 5 | 77 | 1 | 43 | 43 | 7 | 8 |
| A | 200 | 180 | 10 | 77 | 1 | 68 | 71 | 16 | 19 |
| A | 200 | 180 | 15 | 77 | 1 | 84 | 86 | 22 | 27 |
| A | 200 | 180 | 20 | 77 | 1 | 93 | 93 | 24 | 31 |
| A | 200 | 180 | 10 | 90 | 0,67 | 74 | 89 | 13 | 25 |
| A | 200 | 180 | 15 | 90 | 0,67 | 89 | 97 | 17 | 29 |

| | | | | | | | | | |
|---|---|---|---|---|---|---|---|---|---|
| Kat.: Katalysator Temp.: Temperatur im Reaktor MV: Molverhältnis im Feed Sel.: Selektivität (mol%) Umsatz: mol% H EOEtPI P: 2-(2-Hydroxy-ethoxy)-ethyl-piperazin | | | | | | | | | |

### 5. Aufarbeitung

Die Aufarbeitung kann bevorzugt durch die folgenden fünf Schritte erfolgen (hier am Beispiel einer Umsetzung von DEOA mit Monomethylamin oder Monoethylamin):
1) Abtrennung von unumgesetztem primären Amin (Monomethylamin bzw. Monoethylamin) und Rückführung in dem Reaktor
   Ggf. Ausschleusung eines Teils des Monomethylamins bzw. Monoethylamins vom Kopf der Kolonne.
2) Abtrennung von Wasser
3) Abtrennung von leicht siedenden Nebenkomponenten
4) Reindestillation des N-Alkyl-piperazins I über Kopf, dabei Abtrennung von hoch siedenden Nebenkomponenten über Sumpf.
5) Ggf. Rückführung eines Teils der hochsiedenden Nebenkomponenten, insbesondere Diethanolamin, N-(N'-Methyl-2-aminoethyl)-ethanolamin, N-Methyl-N-(2-Aminoethyl)-ethanolamin (bzw. N-(N'-Ethyl-2-aminoethyl)-ethanolamin, N-Ethyl-N-(2-Aminoethyl)-ethanolamin), in die Umsetzung.

## Patentansprüche

1. Verfahren zur Herstellung eines Mono-N-alkyl-piperazins der Formel I 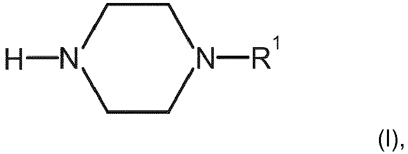 in der R¹ C₁- bis C₅-Alkyl oder 2-(2-Hydroxy-ethoxy)-ethyl bedeutet, durch Umsetzung von Diethanolamin (DEOA) der Formel II 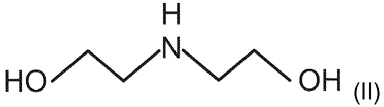 mit einem primären Amin der Formel H₂N-R¹ (III) in Gegenwart von Wasserstoff und eines Katalysator-Formkörpers, **dadurch gekennzeichnet, dass** man die Umsetzung in der Flüssigphase bei einem Absolutdruck im Bereich von 150 bis 250 bar durchführt und die Aminierung mittels eines Katalysator-Formkörpers, dessen Vorläufer gemäß einem Verfahren herstellbar ist, in dem
(i) ein oxidisches Material, umfassend Kupferoxid, Aluminiumoxid und Lanthanoxid bereitgestellt wird,
(ii) dem oxidischen Material pulverförmiges metallisches Kupfer und/oder Kupferblättchen und optional Graphit zugegeben wird,
(iii) das aus Schritt ii resultierende Gemisch zu einem Formkörper verformt wird, durchgeführt wird,
wobei das oxidische Material erhältlich ist durch simultanes oder nacheinander erfolgendes Fällen der Komponente Kupferoxid, der Komponente Aluminiumoxid und der Komponente Lanthanoxid und anschließendes Trocknen und Calcinieren und nach der Verformung nach Schritt iii der Katalysator-Formkörper nochmals calciniert wird.

2. Verfahren nach Anspruch 1, **dadurch gekennzeichnet, dass** das oxidische Material
(a) Kupferoxid mit einem Anteil im Bereich von 50 ≤ x ≤ 80 Gew.-%, berechnet als CuO,
(b) Aluminiumoxid mit einem Anteil im Bereich von 15 ≤ y ≤ 35 Gew.-% und
(c) Lanthanoxid mit einem Anteil im Bereich von 2 ≤ z ≤ 20 Gew.-%,
jeweils bezogen auf das Gesamtgewicht des oxidischen Materials nach Calcinierung, wobei gilt: 80 ≤ x + y + z ≤ 100, insbesondere 95 ≤ x + y + z ≤ 100, umfasst.

3. Verfahren nach Anspruch 1, **dadurch gekennzeichnet, dass** das oxidische Material
(a) Kupferoxid mit einem Anteil im Bereich von 55 ≤ x ≤ 75 Gew.-%, berechnet als CuO,
(b) Aluminiumoxid mit einem Anteil im Bereich von 20 ≤ y ≤ 30 Gew.-% und
(c) Lanthanoxid mit einem Anteil im Bereich von 3 ≤ z ≤ 15 Gew.-%,
jeweils bezogen auf das Gesamtgewicht des oxidischen Materials nach Calcinierung, wobei gilt: 80 ≤ x + y + z ≤ 100, insbesondere 95 ≤ x + y + z ≤ 100, umfasst.

4. Verfahren nach einem der Ansprüche 1 bis 3, **dadurch gekennzeichnet, dass** in Schritt ii Graphit in Mengen im Bereich von 0,5 bis 5 Gew.-%, bezogen auf das Gesamtgewicht des oxidischen Materials nach Calcinierung, zugesetzt wird.

5. Verfahren nach einem der vorhergehenden Ansprüche, **dadurch gekennzeichnet, dass** pulverförmiges Kupfer und/oder die Kupferblättchen zusammengenommen in Mengen im Bereich von 0,5 bis 40 Gew.-%, bezogen auf das Gesamtgewicht des oxidischen Materials nach Calcinierung, zugesetzt werden.

6. Verfahren nach einem der vorhergehenden Ansprüche, **dadurch gekennzeichnet, dass** dem aus Schritt ii resultierenden Gemisch vor der Verformung in Schritt iii 0,5 bis 5 Gew.-% Graphit, bezogen auf das Gesamtgewicht des aus Schritt ii resultierenden Gemisches, zugegeben wird.

7. Verfahren nach einem der vorhergehenden Ansprüche, **dadurch gekennzeichnet, dass** die Summe der Anteile aus oxidischem Material, metallischem Kupferpulver und/oder Kupferplättchen und ggf. Graphit mindestens 95 Gew.-% des Katalysator-Formkörpers ergibt.

8. Verfahren nach einem der vorhergehenden Ansprüche, **dadurch gekennzeichnet, dass** der Katalysator-Formkörper kein Rhenium und/oder Ruthenium enthält.

9. Verfahren nach einem der vorhergehenden Ansprüche, **dadurch gekennzeichnet, dass** der Katalysatorformkörper kein Eisen und/oder Zink enthält.

10. Verfahren nach einem der vorhergehenden Ansprüche, **dadurch gekennzeichnet, dass** der Katalysatorformkörper keine sauerstoffhaltigen Verbindungen des Siliziums und/oder des Zirkoniums und/oder des Titans enthält.

11. Verfahren nach einem der vorhergehenden Ansprüche, **dadurch gekennzeichnet, dass** man die Umsetzung bei einer Temperatur im Bereich von 180 bis 240 °C durchführt.

12. Verfahren nach einem der vorhergehenden Ansprüche, **dadurch gekennzeichnet, dass** man die Umsetzung bei einem Absolutdruck im Bereich von 160 bis 220 bar durchführt.

13. Verfahren nach einem der vorhergehenden Ansprüche, **dadurch gekennzeichnet, dass** das primäre Amin III in der 5- bis 15-fachen molaren Menge bezogen auf das eingesetzte DEOA eingesetzt wird.

14. Verfahren nach einem der vorhergehenden Ansprüche 1 bis 12, **dadurch gekennzeichnet, dass** Aminodiglykol (ADG) in der 0,2- bis 2-fachen molaren Menge bezogen auf das eingesetzte DEOA eingesetzt wird.

15. Verfahren nach einem der vorhergehenden Ansprüche 1 bis 12, **dadurch gekennzeichnet, dass** Monomethylamin (MMA) in der 4- bis 13-fachen molaren Menge bezogen auf das eingesetzte DEOA eingesetzt wird.

16. Verfahren nach einem der vorhergehenden Ansprüche 1 bis 12, **dadurch gekennzeichnet, dass** Monoethylamin (MEA) in der 2- bis 10-fachen molaren Menge bezogen auf das eingesetzte DEOA eingesetzt wird.

17. Verfahren nach einem der vorhergehenden Ansprüche, **dadurch gekennzeichnet, dass** der Katalysator im Reaktor als Festbett angeordnet ist.

18. Verfahren nach einem der vorhergehenden Ansprüche, **dadurch gekennzeichnet, dass** es kontinuierlich durchgeführt wird.

19. Verfahren nach den beiden vorhergehenden Ansprüchen, **dadurch gekennzeichnet, dass** man die Umsetzung zunächst bei einer Temperatur im Bereich von 80 bis 160 °C und danach bei einer Temperatur im Bereich von 180 bis 240 °C durchführt.

20. Verfahren nach einem der drei vorhergehenden Ansprüche, **dadurch gekennzeichnet, dass** die Umsetzung in einem Rohrreaktor erfolgt.

21. Verfahren nach einem der vier vorhergehenden Ansprüche, **dadurch gekennzeichnet, dass** die Umsetzung bei einer Kreisgasfahrweise erfolgt.

22. Verfahren nach einem der vorhergehenden Ansprüche, **dadurch gekennzeichnet, dass** man das DEOA als wässrige Lösung einsetzt.

23. Verfahren nach einem der vorhergehenden Ansprüche, **dadurch gekennzeichnet, dass** man das primäre Amin III als wässrige Lösung einsetzt.

24. Verfahren nach einem der vorhergehenden Ansprüche, **dadurch gekennzeichnet, dass** man die Umsetzung bei einer Katalysatorbelastung im Bereich von 0,3 bis 0,7 kg DEOA / (I_{Kat}. ● h) durchführt.

25. Verfahren nach einem der vorhergehenden Ansprüche, **dadurch gekennzeichnet, dass** man die Umsetzung bei einer Katalysatorbelastung im Bereich von 400 bis 1400 Normliter Wasserstoff / (I_{Kat}. ● h) durchführt.

26. Verfahren nach einem der vorhergehenden Ansprüche zur Herstellung eines Mono-N-alkyl-piperazins der Formel I, in der R¹ Methyl, Ethyl oder 2-(2-Hydroxy-ethoxy)-ethyl bedeutet, durch Umsetzung von Diethanolamin (DEOA) der Formel II mit einem primären Amin der Formel H₂N-R¹ (III).

27. Verfahren nach einem der vorhergehenden Ansprüche, **dadurch gekennzeichnet, dass** aus dem Reaktionsprodukt der Umsetzung durch Destillation
(i) zunächst ggf. unumgesetztes primäres Amin III über Kopf abgetrennt wird,
(ii) Wasser über Kopf abgetrennt wird,
(iii) ggf. vorhandene Nebenprodukte mit einem niedrigeren Siedepunkt als dem des Verfahrensprodukts I über Kopf abgetrennt werden,
(iv) das Verfahrensprodukt Mono-N-alkyl-piperazin I über Kopf abgetrennt wird, wobei ggf. vorhandene Nebenprodukte mit einem höheren Siedepunkt als dem des Verfahrensprodukts I und ggf. vorhandenes unumgesetztes DEOA (II) im Sumpf verbleiben.

28. Verfahren nach dem vorhergehenden Anspruch, **dadurch gekennzeichnet, dass** durch Destillation
(v) aus dem Sumpf des Schrittes iv ggf. vorhandenes unumgesetztes DEOA (II) und/oder ggf. vorhandenes Alkylaminoethylethanolamin als Nebenprodukt mit der Formel IV 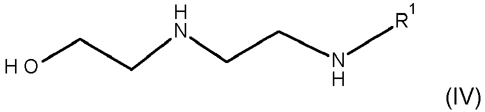 über Kopf abgetrennt und in die Umsetzung zurückgeführt werden.

29. Verfahren nach einem der beiden vorhergehenden Ansprüche, **dadurch gekennzeichnet, dass** in Schritt i abgetrenntes primäres Amin III mit einer Reinheit von 90 bis 99,9 Gew.-% in die Umsetzung zurückgeführt wird, wobei bevorzugt ein Teil des Amins III ausgeschleust wird.

30. Verfahren nach einem der Ansprüche 1 bis 26 zur Herstellung von Mono-N-alkyl-piperazin der Formel I mit R¹ = 2-(2-Hydroxy-ethoxy)-ethyl, **dadurch gekennzeichnet, dass** aus dem Reaktionsprodukt der Umsetzung durch Destillation
(i) zunächst Wasser über Kopf abgetrennt wird,
(ii) ggf. unumgesetztes primäres Amin III (= ADG) über Kopf abgetrennt wird,
(iii) ggf. vorhandene Nebenprodukte mit einem niedrigeren Siedepunkt als dem des Verfahrensprodukts I über Kopf abgetrennt werden,
(iv) das Verfahrensprodukt Mono-N-alkyl-piperazin I über Kopf abgetrennt wird, wobei ggf. vorhandene Nebenprodukte mit einem höheren Siedepunkt als dem des Verfahrensprodukts I und ggf. vorhandenes unumgesetztes DEOA (II) im Sumpf verbleiben.

31. Verfahren nach dem vorhergehenden Anspruch, **dadurch gekennzeichnet, dass** durch Destillation
(v) aus dem Sumpf des Schrittes iv ggf. vorhandenes unumgesetztes DEOA (II) und/oder ggf. vorhandenes Alkylaminoethylethanolamin als Nebenprodukt mit der Formel IV 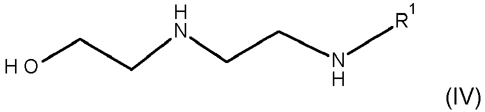 über Kopf abgetrennt und in die Umsetzung zurückgeführt werden.

32. Verfahren nach einem der beiden vorhergehenden Ansprüche, **dadurch gekennzeichnet, dass** in Schritt ii abgetrenntes ADG mit einer Reinheit von 90 bis 99,9 Gew.-% in die Umsetzung zurückgeführt wird, wobei bevorzugt ein Teil des ADGs ausgeschleust wird.

## Claims

1. A process for the preparation of a mono-N-alkylpiperazine of the formula I 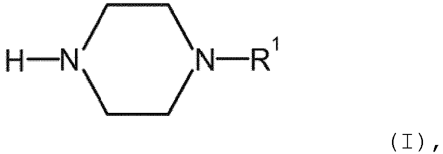 in which R¹ is C₁- to C₅-alkyl or 2-(2-hydroxyethoxy)ethyl,
by reacting diethanolamine (DEOA) of the formula II 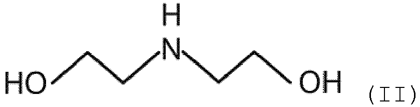 with a primary amine of the formula H₂N-R¹ (III) in the presence of hydrogen and a catalyst molding, wherein the reaction is carried out in the liquid phase at an absolute pressure in the range from 150 to 250 bar and the amination is carried out by means of a catalyst molding, the precursor of which can be prepared according to a process in which
(i) an oxidic material comprising copper oxide, aluminum oxide and lanthanum oxide is provided,
(ii) pulverulent metallic copper and/or copper flakes and optionally graphite is added to the oxidic material,
(iii) the mixture resulting from step ii is shaped to give a molding,
where the oxidic material is obtainable by simultaneous or successive precipitation of the component copper oxide, of the component aluminum oxide and of the component lanthanum oxide and subsequent drying and calcination
and, after the shaping according to step iii, the catalyst molding is calcined again.

2. The process according to claim 1, wherein the oxidic material comprises
(a) copper oxide with a fraction in the range from 2 ≤ x ≤ 80% by weight, calculated as CuO,
(b) aluminum oxide with a fraction in the range from 15 ≤ y ≤ 35% by weight and
(c) lanthanum oxide with a fraction in the range from 2 ≤ z ≤ 20% by weight,
in each case based on the total weight of the oxidic material after calcination, where: 80 ≤ x + y + z ≤ 100, in particular 95 ≤ x + y + z ≤ 100.

3. The process according to claim 1, wherein the oxidic material comprises
(a) copper oxide with a fraction in the range from 2 ≤ x ≤ 75% by weight, calculated as CuO,
(b) aluminum oxide with a fraction in the range from 20 ≤ y ≤ 30% by weight and
(c) lanthanum oxide with a fraction in the range from 3 ≤ z ≤ 15% by weight,
in each case based on the total weight of the oxidic material after calcination, where: 80 ≤ x + y + z ≤ 100, in particular 95 ≤ x + y + z ≤ 100.

4. The process according to any one of claims 1 to 3, wherein, in step ii, graphite is added in amounts in the range from 0.5 to 5% by weight, based on the total weight of the oxidic material after calcination.

5. The process according to any one of the preceding claims, wherein pulverulent copper and/or the copper flakes taken together are added in amounts in the range from 0.5 to 40% by weight, based on the total weight of the oxidic material after calcination.

6. The process according to any one of the preceding claims, wherein 0.5 to 5% by weight of graphite is added to the mixture resulting from step ii prior to the shaping in step iii, based on the total weight of the mixture resulting from step ii.

7. The process according to any one of the preceding claims, wherein the sum of the fractions of oxidic material, metallic copper powder and/or copper flakes and optionally graphite is at least 95% by weight of the catalyst molding.

8. The process according to any one of the preceding claims, wherein the catalyst molding comprises no rhenium and/or ruthenium.

9. The process according to any one of the preceding claims, wherein the catalyst molding comprises no iron and/or zinc.

10. The process according to any one of the preceding claims, wherein the catalyst molding comprises no oxygen-containing compounds of silicon and/or of zirconium and/or of titanium.

11. The process according to any one of the preceding claims, wherein the reaction is carried out at a temperature in the range from 180 to 240°C.

12. The process according to any one of the preceding claims, wherein the reaction is carried out at an absolute pressure in the range from 160 to 220 bar.

13. The process according to any one of the preceding claims, wherein the primary amine III is used in a 5- to 15-fold molar amount, based on the DEOA used.

14. The process according to any one of the preceding claims 1 to 12, wherein aminodiglycol (ADG) is used in a 0.2- to 2-fold molar amount, based on the DEOA used.

15. The process according to any one of the preceding claims 1 to 12, wherein monomethylamine (MMA) is used in a 4- to 13- fold molar amount, based on the DEOA used.

16. The process according to any one of the preceding claims 1 to 12, wherein monoethylamine (MEA) is used in a 2- to 10-fold molar amount, based on the DEOA used.

17. The process according to any one of the preceding claims, wherein the catalyst is arranged as a fixed bed in the reactor.

18. The process according to any one of the preceding claims, which is carried out continuously.

19. The process according to the two preceding claims, wherein the reaction is carried out firstly at a temperature in the range from 80 to 160°C and then at a temperature in the range from 180 to 240°C.

20. The process according to any one of the three preceding claims, wherein the reaction takes place in a tubular reactor.

21. The process according to any one of the four preceding claims, wherein the reaction takes place in a circulating-gas mode.

22. The process according to any one of the preceding claims, wherein the DEOA is used as aqueous solution.

23. The process according to any one of the preceding claims, wherein the primary amine III is used as aqueous solution.

24. The process according to any one of the preceding claims, wherein the reaction is carried out at a catalyst hourly space velocity in the range from 0.3 to 0.7 kg of DEOA / (I_{cat}. ● h).

25. The process according to any one of the preceding claims, wherein the reaction is carried out at a catalyst hourly space velocity in the range from 400 to 1400 liters (stp) of hydrogen/ (I_{cat}. ● h).

26. The process according to any one of the preceding claims for the preparation of a mono-N-alkylpiperazine of the formula I in which R¹ is methyl, ethyl or 2-(2-hydroxyethoxy)ethyl, by reacting diethanolamine (DEOA) of the formula II with a primary amine of the formula H₂N-R¹ (III).

27. The process according to any one of the preceding claims, wherein, from the reaction product of the reaction, by distillation,
(i) firstly optionally unreacted primary amine III is separated off overhead,
(ii) water is separated off overhead,
(iii) optionally present by-products with a lower boiling point than that of the process product I are separated off overhead,
(iv) the process product mono-N-alkylpiperazine I is separated off overhead, with optionally present by-products with a higher boiling point than that of the process product I and optionally present unreacted DEOA (II) remaining in the bottom.

28. The process according to the preceding claim, wherein, by distillation,
(v) from the bottom of step iv, optionally present unreacted DEOA (II) and/or optionally present alkylaminoethylethanolamine as by-product with the formula IV 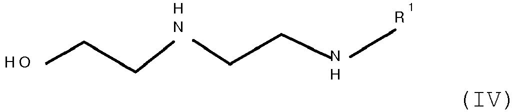 are separated off overhead and returned to the reaction.

29. The process according to either of the two preceding claims, wherein primary amine III separated off in step i and having a purity of from 90 to 99.9% by weight is returned to the reaction, with preferably some of the amine III being removed.

30. The process according to any one of claims 1 to 26 for the preparation of mono-N-alkylpiperazine of the formula I where R'=2-(2-hydroxyethoxy)ethyl, wherein, from the reaction product of the reaction, by distillation
(i) firstly water is separated off overhead,
(ii) optionally unreacted primary amine III (ADG) is separated off overhead,
(iii) optionally present by-products with a lower boiling point than that of the process product I are separated off overhead,
(iv) the process product mono-N-alkylpiperazine I is separated off overhead, with optionally present by-products with a higher boiling point than that of the process product I and optionally present unreacted DEOA (II) remaining in the bottom.

31. The process according to the preceding claim, wherein, by distillation,
(v) from the bottom of step iv, optionally present unreacted DEOA (II) and/or optionally present alkylaminoethylethanolamine as by-product with the formula IV 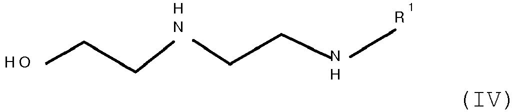 are separated off overhead and returned to the reaction.

32. The process according to either of the two preceding claims, wherein ADG separated off in step ii and having a purity of from 90 to 99.9% by weight is returned to the reaction, with preferably some of the ADG being removed.

## Revendications

1. Procédé de fabrication d'une mono-N-alkyl-pipérazine de formule I 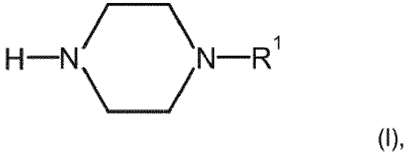 dans laquelle R¹ signifie alkyle en C₁ à C₅ ou 2- (2-hydroxy-éthoxy)-éthyle,
par mise en réaction de diéthanolamine (DEOA) de formule II 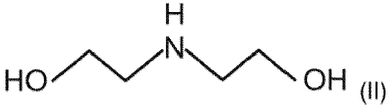 avec une amine primaire de formule H₂N-R¹ (III) en présence d'hydrogène et d'un corps moulé de catalyseur, **caractérisé en ce que** la réaction est réalisée dans la phase liquide à une pression absolue dans la plage allant de 150 à 250 bar et l'amination est réalisée au moyen d'un corps moulé de catalyseur, dont le précurseur peut être fabriqué par un procédé selon lequel
(i) un matériau oxydique, comprenant de l'oxyde de cuivre, de l'oxyde d'aluminium et de l'oxyde de lanthane est préparé,
(ii) du cuivre métallique en poudre et/ou des feuillets de cuivre et éventuellement du graphite sont ajoutés au matériau oxydique,
(iii) le mélange résultant de l'étape ii est façonné en un corps moulé,
le matériau oxydique pouvant être obtenu par précipitation simultanée ou successive du composant oxyde de cuivre, du composant oxyde d'aluminium et du composant oxyde de lanthane, puis séchage et calcination,
et le corps moulé de catalyseur étant encore calciné après le façonnage selon l'étape iii.

2. Procédé selon la revendication 1, **caractérisé en ce que** le matériau oxydique comprend
(a) de l'oxyde de cuivre en une proportion dans la plage de 50 ≤ x ≤ 80 % en poids, calculé sous la forme de CuO,
(b) de l'oxyde d'aluminium en une proportion dans la plage de 15 ≤ y ≤ 35 % en poids, et
(c) de l'oxyde de lanthane en une proportion dans la plage de 2 ≤ z ≤ 20 % en poids,
à chaque fois par rapport au poids total du matériau oxydique après calcination, avec : 80 ≤ x + y + z ≤ 100, notamment 95 ≤ x + y + z ≤ 100.

3. Procédé selon la revendication 1, **caractérisé en ce que** le matériau oxydique comprend
(a) de l'oxyde de cuivre en une proportion dans la plage de 55 ≤ x ≤ 75 % en poids, calculé sous la forme de CuO,
(b) de l'oxyde d'aluminium en une proportion dans la plage de 20 ≤ y ≤ 30 % en poids, et
(c) de l'oxyde de lanthane en une proportion dans la plage de 3 ≤ z ≤ 15 % en poids,
à chaque fois par rapport au poids total du matériau oxydique après calcination, avec : 80 ≤ x + y + z ≤ 100, notamment 95 ≤ x + y + z ≤ 100.

4. Procédé selon l'une quelconque des revendications 1 à 3, **caractérisé en ce qu'**à l'étape ii, du graphite est ajouté en quantités dans la plage allant de 0,5 à 5 % en poids, par rapport au poids total du matériau oxydique après calcination.

5. Procédé selon l'une quelconque des revendications précédentes, **caractérisé en ce que** le cuivre en poudre et/ou les feuillets de cuivre sont ajoutés au total en quantités dans la plage allant de 0,5 à 40 % en poids, par rapport au poids total du matériau oxydique après calcination.

6. Procédé selon l'une quelconque des revendications précédentes, **caractérisé en ce que** 0,5 à 5 % en poids de graphite, par rapport au poids total du mélange résultant de l'étape ii, est ajouté au mélange résultant de l'étape ii avant le façonnage à l'étape iii.

7. Procédé selon l'une quelconque des revendications précédentes, **caractérisé en ce que** la somme des proportions de matériau oxydique, poudre de cuivre métallique et/ou feuillets de cuivre et éventuellement graphite est d'au moins 95 % en poids du corps moulé de catalyseur.

8. Procédé selon l'une quelconque des revendications précédentes, **caractérisé en ce que** le corps moulé de catalyseur ne contient pas de rhénium et/ou de ruthénium.

9. Procédé selon l'une quelconque des revendications précédentes, **caractérisé en ce que** le corps moulé de catalyseur ne contient pas de fer et/ou de zinc.

10. Procédé selon l'une quelconque des revendications précédentes, **caractérisé en ce que** le corps moulé de catalyseur ne contient pas de composés oxygénés de silicium et/ou de zirconium et/ou de titane.

11. Procédé selon l'une quelconque des revendications précédentes, **caractérisé en ce que** la réaction est réalisée à une température dans la plage allant de 180 à 240 °C.

12. Procédé selon l'une quelconque des revendications précédentes, **caractérisé en ce que** la réaction est réalisée à une pression absolue dans la plage allant de 160 à 220 bar.

13. Procédé selon l'une quelconque des revendications précédentes, **caractérisé en ce que** l'amine primaire III est utilisée en une quantité molaire de 5 à 15 fois par rapport à la DEOA utilisée.

14. Procédé selon l'une quelconque des revendications 1 à 12, **caractérisé en ce que** l'aminodiglycol (ADG) est utilisé en une quantité molaire de 0,2 à 2 fois par rapport à la DEOA utilisée.

15. Procédé selon l'une quelconque des revendications 1 à 12, **caractérisé en ce que** la monométhylamine (MMA) est utilisée en une quantité molaire de 4 à 13 fois par rapport à la DEOA utilisée.

16. Procédé selon l'une quelconque des revendications 1 à 12, **caractérisé en ce que** la monoéthylamine (MEA) est utilisée en une quantité molaire de 2 à 10 fois par rapport à la DEOA utilisée.

17. Procédé selon l'une quelconque des revendications précédentes, **caractérisé en ce que** le catalyseur est agencé dans le réacteur sous la forme d'un lit fixe.

18. Procédé selon l'une quelconque des revendications précédentes, **caractérisé en ce qu'**il est réalisé en continu.

19. Procédé selon les deux revendications précédentes, **caractérisé en ce que** la réaction est tout d'abord réalisée à une température dans la plage allant de 80 à 160 °C, puis à une température dans la plage allant de 180 à 240 °C.

20. Procédé selon l'une quelconque des trois revendications précédentes, **caractérisé en ce que** la réaction a lieu dans un réacteur tubulaire.

21. Procédé selon l'une quelconque des quatre revendications précédentes, **caractérisé en ce que** la réaction a lieu en mode de circulation de gaz.

22. Procédé selon l'une quelconque des revendications précédentes, **caractérisé en ce que** la DEOA est utilisée sous la forme d'une solution aqueuse.

23. Procédé selon l'une quelconque des revendications précédentes, **caractérisé en ce que** l'amine primaire III est utilisée sous la forme d'une solution aqueuse.

24. Procédé selon l'une quelconque des revendications précédentes, **caractérisé en ce que** la réaction est réalisée à un chargement du catalyseur dans la plage allant de 0,3 à 0,7 kg de DEOA/(l_{cat}·h).

25. Procédé selon l'une quelconque des revendications précédentes, **caractérisé en ce que** la réaction est réalisée à un chargement du catalyseur dans la plage allant de 400 à 1 400 litres normés d'hydrogène/ (l_{cat}·h).

26. Procédé selon l'une quelconque des revendications précédentes, pour la fabrication d'une mono-N-alkyl-pipérazine de formule I, dans laquelle R¹ signifie méthyle, éthyle ou 2-(2-hydroxy-éthoxy)-éthyle, par mise en réaction de diéthanolamine (DEOA) de formule II avec une amine primaire de formule H₂N-R¹ (III).

27. Procédé selon l'une quelconque des revendications précédentes, **caractérisé en ce qu'**à partir du produit de la réaction, par distillation,
(i) l'amine primaire III éventuellement non réagie est tout d'abord séparée par la tête,
(ii) l'eau est séparée par la tête,
(iii) les produits secondaires d'un point d'ébullition plus faible que celui du produit du procédé I éventuellement présents sont séparés par la tête,
(iv) le produit du procédé mono-N-alkyl-pipérazine I est séparé par la tête, les produits secondaires d'un point d'ébullition plus élevé que celui du produit du procédé I éventuellement présents et la DEOA (II) non réagie éventuellement présente restant dans le fond.

28. Procédé selon la revendication précédente, **caractérisé en ce que**, par distillation,
(v) à partir du fond de l'étape iv, la DEOA (II) non réagie éventuellement présente et/ou l'alkylaminoéthyléthanolamine éventuellement présente en tant que produit secondaire de formule IV 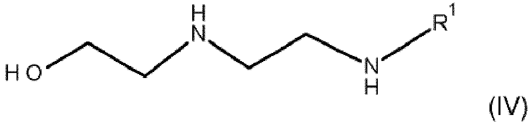 sont séparées par la tête et recyclées dans la réaction.

29. Procédé selon l'une quelconque des deux revendications précédentes, **caractérisé en ce que** l'amine primaire III séparée à l'étape i d'une pureté de 90 à 99,9 % en poids est recyclée dans la réaction, une partie de l'amine III étant de préférence déchargée.

30. Procédé selon l'une quelconque des revendications 1 à 26, pour la fabrication de mono-N-alkyl-pipérazine de formule I avec R¹ = 2-(2-hydroxy-éthoxy)-éthyle, **caractérisé en ce qu'**à partir du produit de la réaction, par distillation,
(i) l'eau est tout d'abord séparée par la tête,
(ii) l'amine primaire III éventuellement non réagie (= ADG) est séparée par la tête,
(iii) les produits secondaires d'un point d'ébullition plus faible que celui du produit du procédé I éventuellement présents sont séparés par la tête,
(iv) le produit du procédé mono-N-alkyl-pipérazine I est séparé par la tête, les produits secondaires d'un point d'ébullition plus élevé que celui du produit du procédé I éventuellement présents et la DEOA (II) non réagie éventuellement présente restant dans le fond.

31. Procédé selon la revendication précédente, **caractérisé en ce que**, par distillation,
(v) à partir du fond de l'étape iv, la DEOA (II) non réagie éventuellement présente et/ou l'alkylaminoéthyléthanolamine éventuellement présente en tant que produit secondaire de formule IV 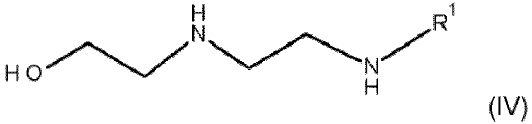 sont séparées par la tête et recyclées dans la réaction.

32. Procédé selon l'une quelconque des deux revendications précédentes, **caractérisé en ce que** l'ADG séparée à l'étape ii d'une pureté de 90 à 99,9 % en poids est recyclée dans la réaction, une partie de l'ADG étant de préférence déchargée.
